# EUROPEAN PATENT APPLICATION

(11) **EP 2 399 590 A1**
(43) Date of publication of application: **28.12.2011**
(21) Application number: 10305683.4
(22) Date of filing: 25.06.2010
(51) Int. Cl.: A61K 31/727, A61P 7/02

(54) **Semuloparin for the prevention of a mortality and/or morbidity event in a patient undergoing major orthopaedic surgery**

(71) Applicant: Aventis Pharma S.A., 92160 Antony (FR)
(72) Inventor: The designation of the inventor has not yet been filed
(74) Representative: Romanowski, Caroline

(57) **Abstract**

The invention relates to the use of semuloparin or a pharmaceutically acceptable salt thereof for the prevention of a mortality and/or morbidity event, more specifically venous thromboembolism and death, in a patient undergoing major orthopaedic surgery, such as hip replacement, knee replacement or hip fracture surgery.

## Description

The invention relates to the use of semuloparin or a pharmaceutically acceptable salt thereof for the prevention of a mortality and/or morbidity event, more specifically venous thromboembolism and death, in a patient undergoing major orthopaedic surgery.

Semuloparin, or AVE5026 (sanofi-aventis laboratory code) belongs to a new generation of hemisynthetic heparins. It is a new ultra-low molecular weight heparin, with an average molecular weight of 2000-3000 Daltons and a novel antithrombotic profile resulting from high anti-Factor Xa activity (- 160 U/mg) and residual anti-Factor IIa activity (- 2 U/mg), as described in Journal of Thrombosis and Haemostasis, 2009, vol. 7, 1143-1151. It is obtained by selective and controlled depolymerization of heparin, as described in the patent application WO 2004/033503 and in J. Thromb Haemost. (ibid). Semuloparin, in the form of its sodium salt, is in clinical development for venous thromboembolism prevention.

At the present time, low-molecular-weight heparins (LMWHs), the synthetic pentasaccharide fondaparinux or dose-adjusted anti-vitamin K are the primary treatments for the prevention of venous thromboembolic diseases.

Patients undergoing surgery are at substantial risk of postoperative venous thromboembolism (hereafter "VTE"). In addition, many hospitalized patients have additional risk factors for VTE. In order to avoid postoperative venous thromboembolic complication, guidelines in thrombosis (ACCP guidelines) recommend the use of antithrombotic drugs for certain categories of surgical patients. Amongst these drugs, the LMWH enoxaparin is the pharmacological VTE prevention agent with the highest clinical documentation in surgical populations and with the largest clinical use in this setting. Enoxaparin has an average molecular weight of 3800-5000 Daltons, an anti-Factor Xa activity comprised between 90 and 125 IU/mg and an anti-Factor IIa activity of 20-35 IU/mg.

The Applicant has now found that a product outside of the LMWH class, namely the ultra-low molecular weight heparin (ULMWH) semuloparin, allows to reduce the occurrence of mortality and/or morbidity events in patients undergoing major orthopaedic surgery.

Therefore, the subject-matter of the invention is semuloparin for use in the prevention of a mortality and/or morbidity event in a patient undergoing major orthopaedic surgery, wherein said event is selected from VTE and death and wherein the efficacy of said use is clinically proven by phase III clinical trials.

According to the present invention, the terms below have the following meanings:
- "major orthopedic surgery" refers to hip replacement, hip fracture or knee replacement surgery ;
- "a patient" refers to a patient being at risk of VTE and for whom prophylaxis of venous thromboembolic events is indicated;
- "death" refer to all causes of death;
- "phase III clinical trial" refers to a multicenter, randomized, double-blinded study involving a large patients group (1.000 to more than 2.000), aiming at being the definitive assessment of how effective the drug is, in comparison with current standard treatment.

As used herein, the wording "semuloparin for use in the prevention of ..." shall be understood as being equivalent to the wording "use of semuloparin for the prevention of ..." or "use of semuloparin for the preparation of a medicament for use in the prevention of ...".

According to the instant invention, semuloparin designates the sodium salt of this ULMWH or any other pharmaceutically acceptable salt thereof.

More specifically, the subject-matter of the invention is semuloparin for use in the prevention of a mortality and/or morbidity event in a patient undergoing major orthopaedic surgery, being at risk of VTE and for whom prophylaxis of venous thromboembolic events is indicated, wherein said mortality and/or morbidity event is selected from VTE and death and wherein the efficacy of said use is clinically proven by phase III clinical trials.

In particular, the subject-matter of the invention is semuloparin for its use as described above, wherein said mortality and/or morbidity event is selected from deep vein thrombosis (hereafter "DVT", including proximal or distal DVT), non-fatal pulmonary embolism and death.

In the framework of the present invention, the terms below have the following meanings:
- "deep vein thrombosis" : designates a blood clot in a deep vein of the lower limbs ;
- "proximal DVT" : designates a DVT event occurring above the knee ;
- "distal DVT" : designates a DVT event occurring below the knee.

In another aspect of the instant invention, major orthopaedic surgery is selected from hip replacement surgery, hip fracture surgery or knee replacement surgery.

In another embodiment, the invention relates to semuloparin for use in the prevention of a mortality and/or morbidity event in a patient undergoing major orthopaedic surgery, wherein said event is selected from venous thromboembolism and death, wherein the efficacy of said use is clinically proven by phase III clinical trials, and wherein semuloparin displays a better efficacy compared to a standard antithrombotic treatment.

In particular, said standard antithrombotic treatment is the LMWH enoxaparin.

Indeed, it has been found that administration of semuloparin after major orthopaedic surgery enables to decrease the occurrence of venous thromboembolism and death in the patients having benefited from a treatment with semuloparin, compared to the occurrence of such events in patients having benefited from the standard treatment with enoxaparin. "Treatment" is understood herein as a venous thromboprophylactic treatment.

In this embodiment of the invention, said mortality and/or morbidity event is in particular deep vein thrombosis.

In this embodiment of the invention, said patient is in particular undergoing hip replacement surgery.

In another embodiment, the invention relates to a method for the prevention of a mortality and/or morbidity event in a patient undergoing major orthopaedic surgery, which comprises the administration of an effective dose of semuloparin or a pharmaceutically acceptable salt thereof to a patient in need thereof, wherein said event is selected from venous thromboembolism and death and wherein the efficacy of said use is clinically proven by phase III clinical trials, as described above.

According to the instant invention, semuloparin is administered at a 20 mg daily dose to patients with normal renal function or to patients with mild or moderate renal impairment. For patients with severe renal impairment, semuloparin is administered at a 10 mg daily dose.

According to the instant invention, the renal function of the patients is defined according to estimated creatinine clearance (CLcr) values calculated using the well-known Cockroft-Gault formula, and is classified according to the following characteristics :
- normal renal function: CLcr > 80 mL/min;
- mild renal impairment: 50 ≤ CLcr ≤ 80 mL/min;
- moderate renal impairment: 30 ≤ CLcr < 50 mL/min;
- severe renal impairment: CLcr < 30 mL/min.

The treatment with semuloparin is advantageously administered once daily. As used therein, "daily" means an administration every 24 hours plus or minus 4 hours. Said treatment is advantageously administered for 7 to 10 days.

The invention also relates to the use of semuloparin or a pharmaceutically acceptable salt thereof for the manufacture of a medicament useful for the prevention of a mortality and/or morbidity event in a patient undergoing major orthopaedic surgery, wherein said event is selected from venous thromboembolism and death and wherein the efficacy of said use is clinically proven by phase III clinical trials.

Having now described the present invention, the same will be more clearly understood by reference to the following examples of the invention, which are included herewith for purposes of illustration only and are not intended to be limiting of the invention.

The following abbreviations shall be used:
b.i.d.: *bis in die* (twice daily)
CLcr: Creatinine Clearance
DVT: deep vein thrombosis
IP: investigational product
PE: pulmonary embolism
UFH: unfractionated heparin
LMWH: low molecular weight heparin
OR: Odds Ratio
q.d.: *quaque die* (once daily)
s.c.: subcutaneously
SRI: severe renal impairment
VTE: venous thromboembolism
95% exact CI: 95% exact Confidence Interval
95% mid-p CI: 95% mid-p Confidence Interval

### Example 1): Preparation of semuloparin

Semuloparin, in the form of a sodium salt, is obtained by a chemoselective depolymerization of heparin, activated through its benzyl ester derivative, by the phosphazene base 2-tert-butylimino-2-diethylamino-1,3-dimethylperhydro-1,2,3-diazaphosphorine (BEMP). The hemisynthetic pathway, allowing to recover semuloparin in the form of a sodium salt, is described in the patent application WO 2004/033503 and in Journal of Thrombosis and Haemostasis, 2009, vol. 7, 1143-1151. This procedure yields semuloparin with an average molecular weight around 2400 Da, an anti-Factor Xa activity of - 160 U/mg and a residual anti-Factor IIa activity (~2 U/mg).

**Example 2): The SAVE-KNEE study.** A Multinational, Multicenter, Randomized, Double Blind Study comparing the Efficacy and Safety of AVE5026 with enoxaparin for the Prevention of Venous Thromboembolism in Patients Undergoing Elective Knee Replacement Surgery.

### 1) Study objectives

The primary objective of the study is to compare the efficacy of once daily (q.d.) subcutaneous (s.c.) injections of 20 mg AVE5026 (10 mg for SRI patients) with twice daily (b.i.d.) s.c. injections of 30 mg enoxaparin (20 mg q.d. for SRI patients) administered during 7-10 days after surgery for the prevention of venous thromboembolic events in patients undergoing elective knee replacement surgery. The secondary objectives of this study are to evaluate the safety of AVE5026 in patients undergoing elective knee replacement surgery and to document AVE5026 exposures in this population.

### 2) Study design

Patient's eligibility is determined during the screening period (within the 2 weeks prior to surgery) and is reviewed before randomization.

Randomized treatment is allocated to eligible patients, taking into account the geographical region of the patient and the estimated CLcr at baseline (< or ≥ 30 mL/min).

An end of treatment visit is performed the day of last IP injection or at Day 10, whichever comes first. A bilateral venography is performed between Day 7 and Day 11. A follow-up visit is scheduled at Day 35-42. Maximum duration of study participation is therefore 42 days, including a treatment period up to Day 7-10 and a follow-up period with a visit at Day 35-42.

### 3) Patients

A total of 1150 patients are randomized in the study.

Patients meeting the following criteria are suitable for enrolment in the study:
- Elective knee replacement surgery or a revision of at least one component of a previously implanted knee prosthesis performed ≥ 6 months prior to study entry.
- Signed written informed consent.

Patients meeting one of the following criteria are excluded from enrolment into the study:
1. Legal lower age limitations (country specific).
2. Any major orthopedic surgery in the 3 months prior to study start.
3. Elective knee surgery with polyethylene liner exchange only.
4. First step of a two-step exchange arthroplasty for infection after knee replacement.
5. Hemostasis prior to the first IP injection not established
6. Clinical signs or symptoms of DVT or PE within the last 12 months or known post phlebitic syndrome.
7. Known sensitivity to iodine or contrast dyes, and any contra-indications to the performance of venography.
8. Any treatment or procedure within 2 weeks prior to randomization, or planned during the course of the study treatment period, that could affect the incidence of VTE, such as:
   - Parenteral anticoagulants (UFH, LMWH [eg, enoxaparin, dalteparin, nadroparin], fondaparinux, bivalirudin, hirudin)
   - Oral anticoagulants (vitamin K antagonists)
   - GPIIb/IIIa antagonists: abciximab, eptifibatide, tirofiban
   - Thrombolytic agents
   - Dextrans
   - Intermittent pneumatic compression of the legs (IPC)
9. Known progressive malignant disease.
10. Subject unlikely to comply with protocol (eg, uncooperative attitude, inability to return for follow-up visits, inability to receive daily injection by a Health Care Professional after hospital discharge and unlikelihood of completing the study).
11. Treatment with any investigational product or investigational device in the last 30 days or 5 half lives (whichever is longer) prior to randomization.
12. Any previous exposure to AVE5026
13. Active major bleeding.
14. Thrombocytopenia associated with a positive in vitro test for anti-platelet antibody in the presence of enoxaparin sodium.
15. Known hypersensitivity to enoxaparin sodium (eg, pruritus, urticaria, anaphylactoid reactions).
16. Known hypersensitivity to heparin or pork products.
17. Conditions with increased risk of hemorrhage, such as bacterial endocarditis, congenital or acquired bleeding disorders, active ulcerative and angiodysplastic gastrointestinal disease, hemorrhagic stroke, or shortly after brain, spinal, or ophthalmological surgery.
18. End stage renal disease (estimated creatinine clearance <10 mL/min) or patient on dialysis.
19. Pregnant or breast-feeding women.
20. Women of childbearing potential not protected by highly effective contraceptive method of birth control as defined for contraception in the Informed Consent Form for the duration of the study and/or who are unwilling or unable to be tested for pregnancy.

### 4) Treatments

Sanofi-aventis supplies and manufactures the blinded treatments for this study. According to their randomized assignments, patients receive either AVE5026 or enoxaparin. Both treatments are presented as a ready-to-use 0.5 ml prefilled syringe, identical in appearance, and containing the same volume of a sterile, isotonic solution with sodium chloride 0.9 % and water for injection.

The matching placebo syringe is strictly identical in appearance, containing the same volume but without active component.

AVE5026 or its placebo are administered subcutaneously. The entire volume of the pre-filled syringe must be injected.

Investigational Product (IP) is administered in a blind manner for 7 to 10 days after surgery (Day 1 is the day of surgery). Patients receive either:
- Enoxaparin, 30 mg sc b.i.d. (20 mg q.d. for patients with SRI, defined as estimated CLcr < 30 mL/min), or
- AVE5026, 20 mg sc q.d. (10 mg q.d. for patients with SRI).

### 5) Results

The efficacy analysis period is defined as the period from randomization up to Day 11 (Day 1 being the day of surgery) or up to the mandatory bilateral venography, whichever comes first.

The primary efficacy population includes all randomized patients who received at least one IP injection (active or placebo), who underwent elective knee replacement surgery and with a non-missing primary efficacy endpoint.

The primary analysis compares the two groups (semuloparin and enoxaparin) using an exact 2-sided stratified test at a α level of 0.05 (Gart 1970). Event rates per treatment group are summarized.

Tables 1 and 2 describe the primary efficacy analysis of the SAVE-KNEE study.

**Table 1: Any VTE or death during the efficacy analysis period**

| | Semuloparin (N = 428) | Enoxaparin (N = 427) |
|---|---|---|
| Any VTE or death: | | |
| n (%) | 105 (24.5%) | 120(28.1%) |
| Comparison versus enoxaparin: | | |
| . Common OR (95% exact CI) | | 0.83 (0.60 to 1.14) |
| . p-value | | 0.2440 |

| | | |
|---|---|---|
| N: number of patients in the primary efficacy population n: number of patients showing a given event | | |

**Table 2: Components of the primary efficacy endpoint**

| | Semuloparin | Enoxaparin | OR (95% mid-p CI) |
|---|---|---|---|
| Any DVT | | | |
| n/N (%) | 105/428 (24.5%) | 120/427 (28.1%) | 0.83 (0.61 to 1.13) |
| Any proximal DVT | | | |
| n/N (%) | 17/484 (3.5%) | 10/487 (2.1 %) | 1.74 (0.79 to 3.98) |
| Distal DVT only | | | |
| n/N (%) | 84/425 (19.8%) | 103/424 (24.3%) | 0.77 (0.55 to 1.06) |
| Non-fatal PE | | | |
| n/N (%) | 0/573 (0%) | 1/538 (0.2%) | 0.00 (0.00 to 18.83) |
| All cause death | | | |
| n/N (%) | 0/573 (0%) | 0/568 (0%) | NA |

| | | | |
|---|---|---|---|
| N: number of efficacy evaluable patients n: number of patients showing a given event NA: non applicable | | | |

These results suggest that semuloparin has similar efficacy as enoxaparin for the prevention of VTE and death (DVT, non-fatal PE and death) in patients undergoing elective knee replacement surgery.

**Example 3): The SAVE-HIP1 study.** A Multinational, Multicenter, Randomized, Double Blind Study comparing the Efficacy and Safety of AVE5026 with enoxaparin for the Prevention of Venous Thromboembolism in Patients Undergoing Elective Total Hip Replacement Surgery.

### 1) Study objectives

The primary objective of the study is to compare the efficacy of once daily (q.d.) subcutaneous (s.c.) injections of 20 mg AVE5026 (10 mg for patients with SRI) with q.d. s.c. injections of 40 mg enoxaparin (20 mg for patients with SRI) administered during 7-10 days after surgery for the prevention of venous thromboembolic events in patients undergoing elective hip replacement surgery. The secondary objectives of this study are to evaluate the safety of AVE5026 in patients undergoing elective total hip replacement surgery and to document AVE5026 exposures in this population.

### 2) Study design

Patient's eligibility is determined during the screening period (within the 2 weeks prior to surgery) and is reviewed before randomization (day before surgery or day of surgery).

Randomized treatment is allocated to eligible patients, taking into account the geographical region of the patient, the timing of the first IP injection and the estimated CLcr at screening (< or ≥ 30 mL/min).

An end of treatment visit is performed the day of last IP injection or at Day 10, whichever comes first. A bilateral venography is performed between Day 7 and Day 11. A follow-up visit is scheduled at Day 35-42.

Maximum duration of study participation is therefore 42 days, including a treatment period up to Day 7-10 and a follow-up period with a visit at Day 35-42.

### 3) Patients

A total of 2326 patients are randomized in the study.

Patients meeting the following criteria are suitable for enrolment in the study:
- Elective hip replacement surgery or a revision of at least one component of a previously implanted total hip prosthesis performed ≥ 6 months prior to study entry;
- Signed written informed consent.

Patients meeting one of the following criteria are excluded from enrolment into the study:
1. Elective hip surgery with polyethylene liner exchange only.
2. First step of a two-step exchange arthroplasty for infection after hip prosthesis replacement.
3. Any of the exclusion criteria numbered as items 1, 2 and 6-20 under example 2 above (SAVE-KNEE study).

### 4) Treatments

Sanofi-aventis supplies and manufactures the blinded treatments for this study. According to their randomized assignments, patients receive either AVE5026 or enoxaparin. Both treatments are presented as a ready-to-use 0.5 ml prefilled syringe, identical in appearance, and containing the same volume of a sterile, isotonic solution with sodium chloride 0.9 % and water for injection.

Regarding the pre-operative IP injection (if planned) and the post-operative IP injections to be administered 8 ± 1 hours and 12 ± 1 hours after the end of the surgery, the corresponding pre-filled syringes contain either active drug or placebo. The matching placebo syringe is strictly identical in appearance, containing the same volume but without active component.

AVE5026 or enoxaparin are administered subcutaneously. The entire volume of the pre-filled syringe must be injected.

Investigational Product (IP) is administered in a blinded manner once daily during 7-10 days after surgery. Patients receive either enoxaparin, or AVE5026. The first pre-operative injection (enoxaparin for patients allocated to comparator group and placebo for patients allocated to AVE5026 group) is administered 12 ± 1 hours before the surgical procedure. The pre-operative injection may be omitted as per local enoxaparin labelling. The first post-operative injection (AVE5026 or placebo) is administered 8 ± 1 hours after incision closure, provided that hemostasis has been established. The second post-operative injection (enoxaparin or placebo) is administered 12 ± 1 hours after incision closure. Then, in any case, patients receive on the following days once daily IP injection (enoxaparin or AVE5026).

In case of no pre-operative injection, the first post-operative injection (AVE5026 or placebo) is administered 8 ± 1 hours after incision closure on Day 1, provided that hemostasis has been established. The second post-operative injection (enoxaparin or placebo) is administered 12 ± 1 hours after incision closure. Then, one daily injection (AVE5026 or enoxaparin) is administered on the following days, in the morning from Day 2 and for 7 to 10 days.

### 5) Results

The efficacy analysis period is defined as the period from randomization up to Day 11 (Day 1 being the day of surgery) or up to the mandatory bilateral venography, whichever comes first.

The primary efficacy population includes all randomized patients who received at least one IP injection (active or placebo), who underwent elective hip replacement surgery and with a non-missing primary efficacy endpoint.

The primary analysis compares the two groups (semuloparin and enoxaparin) using an exact 2-sided stratified test at a α level of 0.05 (Gart 1970). Event rates per treatment group are summarized.

Tables 3 and 4 describe the primary efficacy analysis of the SAVE-HIP1 study.

**Table 3: Any VTE or death during the efficacy analysis period**

| | Semuloparin (N=916) | Enoxaparin (N = 933) |
|---|---|---|
| Any VTE or death: | | |
| n (%) | 58 (6.3%) | 105 (11.3%) |
| Comparison versus enoxaparin: | | |
| . Common OR (95% exact CI) | | 0.53 (0.37 to 0.75) |
| . p-value | | 0.0002 |

| | | |
|---|---|---|
| N: number of patients in the primary efficacy population n: number of patients showing a given event | | |

**Table 4: Components of the primary efficacy endpoint**

| | Semuloparin | Enoxaparin | OR (95% mid-p CI) |
|---|---|---|---|
| Any DVT | | | |
| n/N (%) | 57/915 (6.2%) | 103/931 (11.1 %) | 0.54 (0.38 to 0.75) |
| Any proximal DVT | | | |
| n/N (%) | 13/1002 (1.3%) | 15/1011 (1.5%) | 0.88 (0.41 to 1.87) |
| Distal DVT only | | | |
| n/N (%) | 42/915 (4.6%) | 84/931 (9.0%) | 0.49 (0.33 to 0.71) |
| Non-fatal PE | | | |
| n/N (%) | 0/1150 (0%) | 0/1152 (0%) | NA |
| All cause death | | | |
| n/N (%) | 1 /1150 (<0.1%) | 2/1152 (0.2%) | 0.50 (0.02 to 6.59) |

| | | | |
|---|---|---|---|
| N: number of efficacy evaluable patients n: number of patients showing a given event NA: non applicable | | | |

These results demonstrate that semuloparin is effective in preventing VTE and death (DVT, non-fatal PE and death) in patients undergoing elective total hip replacement surgery. These results further demonstrate that semuloparin displays better efficacy than enoxaparin for the prevention of VTE and death, in particular for the prevention of DVT (more specifically distal DVT) in those patients.

**Example 4): The SAVE-HIP2 study.** A Multinational, Multicenter, Randomized, Double Blind Study comparing the Efficacy and Safety of AVE5026 with enoxaparin for the Prevention of Venous Thromboembolism in Patients Undergoing Hip Fracture Surgery.

### 1) Study objectives

The primary objective of the study is to compare the efficacy of once daily (q.d.) subcutaneous (s.c.) injections of 20 mg AVE5026 (10 mg for patients with SRI) with q.d. s.c. injections of 40 mg enoxaparin (20 mg for patients with SRI) administered for 7-10 days after surgery for the prevention of venous thromboembolic events in patients undergoing hip fracture surgery. The secondary objectives of this study are to evaluate the safety of AVE5026 in patients undergoing hip fracture surgery and to document AVE5026 exposures in this population.

### 2) Study design

Patient's eligibility is determined at the admission:
- a patient is eligible for a pre-operative randomization if a pre-operative injection of enoxaparin is requested as per local labelling. In this case, this pre-operative IP injection (enoxaparin in the comparator group and placebo in the AVE5026 group) is administered 12 ± 1 hours before start of surgery: .
- a patient is eligible for a post-operative randomization if the post-operative hemostasis has been established just prior to the first IP injection that will be administered 8 ± 1 hours after the end of surgery.

Randomized treatment is allocated to eligible patients, taking into account the geographical region of the patient, the timing of the first IP injection and the estimated CLcr at screening (< or ≥ 30 mL/min).

An end of treatment visit is performed the day of last IP injection or at Day 10, whichever comes first. A bilateral venography is performed between Day 7 and Day 11. A follow-up visit is scheduled at Day 35-42.

Maximum duration of study participation is therefore 42 days, including a treatment period up to Day 7-10 and a follow-up period with a visit at Day 35-42 after randomization.

### 3) Patients

A total of 1003 patients are randomized in the study.

Patients meeting the following criteria are suitable for enrolment in the study:
1. Standard surgery for fracture of the upper third of the femur, including femoral head and neck:
   either planned within the first 36 hours* after admission to hospital, and in the 12 ± 1 hours following the pre-operative IP injection,
   or just performed 8 ± 1 hours previously (from the time of incision closure), and provided that hemostasis has been established.
      * If time from injury to hospital admission is documented to be less than 24 hours, this 36 hours time window can be expanded but in all cases the time from injury to surgery must not exceed 60 hours.
2. Signed written informed consent.

Patients meeting one of the following criteria are excluded from enrolment into the study:
1. Estimated time of injury/fracture >24 hours before admission to hospital.
2. Time from injury/fracture to surgery >60 hours.
3. Multiple trauma affecting more than one organ system.
4. Any of the exclusion criteria numbered as items 1, 2 and 6-20 described under example 2 above (SAVE-KNEE study).

### 4) Treatments

It is proceeded in the same manner as described in example 3 above (SAVE-HIP1 study).

### 5) Results

The efficacy analysis period is defined as the period from randomization up to Day 11 (Day 1 being the day of surgery) or up to the mandatory bilateral venography, whichever comes first.

The primary efficacy population includes all randomized patients who received at least one IP injection (active or placebo), who underwent elective hip fracture surgery and with a non-missing primary efficacy endpoint.

The primary analysis compares the two groups (semuloparin and enoxaparin) using an exact 2-sided stratified test at a α level of 0.05 (Gart 1970). Event rates per treatment group are summarized.

Tables 5 and 6 describe the primary efficacy analysis of the SAVE-HIP2 study.

**Table 5: Any VTE or death during the efficacy analysis period**

| | Semuloparin (N = 384) | Enoxaparin (N = 369) |
|---|---|---|
| Any VTE or death: | | |
| n (%) | 68(17.7%) | 81 (22.0%) |
| Comparison versus enoxaparin: | | |
| . Common OR (95% exact CI) | | 0.77 (0.53 to 1.12) |
| p-value | | 0.1699 |

| | | |
|---|---|---|
| N: number of patients in the primary efficacy population n: number of patients showing a given event | | |

**Table 6: Components of the primary efficacy endpoint**

| | Semuloparin | Enoxaparin | OR (95% mid-p CI) |
|---|---|---|---|
| Any DVT | | | |
| n/N (%) | 63/379 (16.6%) | 79/367 (21.5%) | 0.73 (0.50 to 1.05) |
| Any proximal DVT | | | |
| n/N (%) | 13/426 (3.1%) | 26/420 (6.2%) | 0.48 (0.23 to 0.93) |
| Distal DVT only | | | |
| n/N (%) | 49/378 (13.0%) | 48/362 (13.3%) | 0.97 (0.63 to 1.50) |
| Non-fatal PE | | | |
| n/N (%) | 1/488 (0.2%) | 0/499 (0%) | NA |
| All cause death | | | |
| n/N (%) | 4/488 (0.8%) | 2/499 (0.4%) | 2.05 (0.36 to 16.08) |

| | | | |
|---|---|---|---|
| N: number of efficacy evaluable patients n: number of patients showing a given event NA: non applicable | | | |

These results suggest that semuloparin has similar efficacy as enoxaparin for the prevention of VTE and death (DVT, non-fatal PE and death) in patients having undergone hip fracture surgery

### Example 5: Meta-analysis of the SAVE-KNEE, SAVE-HIP-1 and SAVE-HIP-2 orthopaedic surgery results

A meta-analysis of the three studies described in examples 2, 3 and 4 above was performed using a fixed-effect logistic regression model adjusted on study. Of 4479 patients randomized, 3457 (77.2%) were evaluable for the primary efficacy analysis. The occurrence of the primary end-point of any VTE or all-cause death was significantly lower for semuloparin *versus* enoxaparin, as shown in table 7 below.

**Table 7: Any VTE or death during the efficacy analysis period**

| | Semuloparin (N = 1728) | Enoxaparin (N = 1729) |
|---|---|---|
| Any VTE or death: | | |
| n (%) | 231 (13.4%) | 305 (17.6%) |
| Comparison versus enoxaparin: | | |
| . Common OR (95% exact CI) | | 0.70 (0.58 to 0.85) |
| . p-value | | 0.0003 |

| | | |
|---|---|---|
| N: number of patients in the primary efficacy population n: number of patients showing a given event | | |

**Table 8: Components of the primary efficacy endpoint**

| | Semuloparin | Enoxaparin | OR (95% mid-p CI) |
|---|---|---|---|
| Any DVT | | | |
| n/N (%) | 225/1722 (13.1%) | 301/1725 (17.4%) | 0.69 (0.57 to 0.84) |
| Any proximal DVT | | | |
| n/N (%) | 43/1912 (2.2%) | 51/1918 (2.7%) | 0.84 (0.55 to 1.26) |
| Distal DVT only | | | |
| n/N (%) | 175/1718 (10.2%) | 234/1716 (13.6%) | 0.70 (0.57 to 0.87) |
| Non-fatal PE | | | |
| n/N (%) | 1 /2211 (<0.1%) | 1 /2219 (<0.1%) | 1.01 (0.06 to 16.12) |
| All cause death | | | |
| n/N (%) | 5/2211 (0.2%) | 4/2219 (0.2%) | 1.27 (0.34 to 4.75) |

| | | | |
|---|---|---|---|
| N: number of efficacy evaluable patients n: number of patients showing a given event | | | |

These results demonstrate that semuloparin is effective in preventing VTE and death (DVT, non-fatal PE and death), in patients undergoing major orthopaedic surgery. These results further demonstrates the better efficacy of semuloparin compared to enoxaparin for the prevention of VTE and death in this setting.

## Claims

1. Semuloparin or a pharmaceutically acceptable salt thereof, for use in the prevention of a mortality and/or morbidity event in a patient undergoing major orthopaedic surgery, wherein said event is selected from venous thromboembolism and death and wherein the efficacy of said use is clinically proven by phase III clinical trials.

2. Semuloparin or a pharmaceutically acceptable salt thereof for the use according to claim 1, for use in the prevention of venous thromboembolism, including deep vein thrombosis, non-fatal pulmonary embolism and death.

3. Semuloparin or a pharmaceutically acceptable salt thereof for the use according to claim 1 or claim 2, for use in the prevention of deep vein thrombosis.

4. Semuloparin or a pharmaceutically acceptable salt thereof for the use according to claim 3, for use in the prevention of proximal deep vein thrombosis.

5. Semuloparin or a pharmaceutically acceptable salt thereof for the use according to claim 3, for use in the prevention of distal deep vein thrombosis.

6. Semuloparin or a pharmaceutically acceptable salt thereof for the use according to any of claims 1 to 5, wherein said major orthopaedic surgery is hip replacement surgery.

7. Semuloparin or a pharmaceutically acceptable salt thereof for the use according to any of claims 1 to 5, wherein said major orthopaedic surgery is hip fracture surgery.

8. Semuloparin or a pharmaceutically acceptable salt thereof for the use according to any of claims 1 to 5, wherein said major orthopaedic surgery is knee replacement surgery.

9. Semuloparin or a pharmaceutically acceptable salt thereof for the use according to claim 1, wherein semuloparin displays a better efficacy compared to a standard antithrombotic treatment.

10. Semuloparin or a pharmaceutically acceptable salt thereof for the use according to claim 9, wherein said standard antithrombotic treatment is enoxaparin.

11. Semuloparin or a pharmaceutically acceptable salt thereof for the use according to claim 9 or claim 10, wherein said mortality and/or morbidity event is deep vein thrombosis.

12. Semuloparin or a pharmaceutically acceptable salt thereof for the use according to any of claims 9 to 11, wherein said mortality and/or morbidity event is distal deep vein thrombosis.

13. Semuloparin or a pharmaceutically acceptable salt thereof for the use according to any of claims 9 to 11, wherein said major orthopaedic surgery is hip replacement surgery.

14. Semuloparin or a pharmaceutically acceptable salt thereof for the use according to any of claims 9 to 11, wherein said mortality and/or morbidity event is proximal deep vein thrombosis and wherein said major orthopaedic surgery is hip fracture surgery.

15. Semuloparin or a pharmaceutically acceptable salt thereof for the use according to any of claims 1 to 14, wherein semuloparin is administered at a 20 mg daily dose to patients with normal renal function or to patients with mild or moderate renal impairment.

16. Semuloparin or a pharmaceutically acceptable salt thereof for the use according to any of claims 1 to 14, wherein semuloparin is administered at a 10 mg daily dose in patients with severe renal impairment.

17. Semuloparin or a pharmaceutically acceptable salt thereof for the use according to any of claims 1 to 16, wherein semuloparin is administered once daily.

18. Semuloparin or a pharmaceutically acceptable salt thereof for the use according to any of claims 1 to 17, wherein semuloparin is administered for 7 to 10 days.
